# EUROPEAN PATENT SPECIFICATION

(11) **EP 4 032 600 B1**
(45) Date of publication and mention of the grant of the patent: **02.04.2025**
(21) Application number: 22152656.9
(22) Date of filing: 21.01.2022
(51) Int. Cl.: B01D 53/30, B01D 53/75, B01D 53/86, B01D 53/88, A61L 9/20, B01D 39/06, B01D 46/44, F24F 8/108, F24F 8/167, F24F 8/22

(54) **AIR PURIFICATION APPARATUS, AND METHOD FOR REMOVING PARTICULATES, VOLATILE ORGANIC COMPOUNDS, AND NITROUS OXIDE**
LUFTREINIGUNGSVORRICHTUNG, UND VERFAHREN ZUR ENTFERNUNG VON PARTIKELN, FLÜCHTIGEN ORGANISCHEN VERBINDUNGEN UND DISTICKSTOFFMONOXID
APPAREIL, ET PROCÉDÉ DE PURIFICATION D'AIR POUR ÉLIMINER LES PARTICULES, LES COMPOSÉS ORGANIQUES VOLATILS ET L'OXYDE NITREUX

(30) Priority: 22.01.2021 US 202163140684 P; 12.11.2021 US 202117525247
(43) Date of publication of application: 27.07.2022
(73) Proprietor: The Boeing Company, Arlington, VA 22202 (US)
(72) Inventor: Trent, Stephen M., Everett, WA (US); Licht, Stephanie Katherine, Everett, WA (US)
(74) Representative: Hoffmann Eitle

(56) References cited:
- EP-A1- 3 643 395
- WO-A1-2018/234633
- CN-A- 103 285 695
- CN-U- 209 330 845
- GB-A- 2 472 509
- GB-A- 2 574 477
- KR-A- 20040 064 760

## Description

### TECHNICAL FIELD

The present disclosure relates generally to air filtration and purifying air. More particularly, the present disclosure relates to the sensing of air quality and the removal of particulates, Volatile Organic Compounds (VOC), and nitrous oxide-containing compounds from air, preferably from air in an enclosed environment, including vehicles, and enclosed terrestrial transportation environments.

### BACKGROUND

Volatile organic compounds (VOC) are a class of organic chemicals characterized by a high vapor pressure at room temperature, typically resulting from a low boiling point. They include non-methane hydrocarbons (NMHC) and oxygenated NMHC (e.g., alcohols, aldehydes, and organic acids). VOCs emanate from off-gassing of foams, plastics, fabrics, and other manufactured products, particularly when they are new, and from the solvents in many cleaning products. VOCs are also produced as byproducts of human metabolic processes. Over 200 VOCs have been identified in human alveolar breath. In a closed environment full of people, such as a passenger aircraft, endogenously produced VOCs dominate.

VOCs cannot be removed by typical air filtration methods such as HEPA filtration. Existing systems to reduce VOC concentration in the cabin environment include activated carbon.

Further air contaminants can include various particulates, and can further include nitrous oxide-containing compounds that can contaminate an air supply, for example, as byproducts of production from reduction in nitrogen-containing compounds, particularly in enclosed environments, for example, due to emissions from cigarette and other tobacco-related products, emissions from e-cigarettes, the combustion of vehicle fuel, etc. that can migrate into, linger in, and otherwise accumulate in regions within and proximate to the enclosed and substantially enclosed environments, etc. Enclosed and substantially enclosed environments can include terrestrial transportation buildings, terrestrial and non-terrestrial rooms, and other substantially enclosed environments.

The Background and Technical Field sections of this document are provided to place aspects of the present disclosure in technological and operational context, to assist those of skill in the art in understanding their scope and utility. Unless explicitly identified as such, no statement herein is admitted as prior art merely by its inclusion in the Background section.

EP 3 643 395 A1, according to its abstract, states a compact, lightweight, low power aircraft air filtration and VOC removal unit enables the removal of VOCs from cabin air in a passenger aircraft. A plurality of baffles having air flow-through airflow spaces are spaced apart along a duct. UV LEDs are mounted on the interior sides of the outermost baffles, and on both sides of all interior baffles. A filter module is disposed between pairs of baffles, and spaced from the baffles sufficiently to illuminate the entirety of both sides. Each filter modules comprises a plurality of filters. The filters are selected from a coarse foam, a fine foam, or a fused quartz filament felt. Each filter is loaded with a catalyst including one or more of AEROXIDE^{®} P25, other pure titanium dioxide (TiO₂), iron-doped TiO₂, carbon-doped TiO₂, and combinations thereof. The catalysts on the filters, under UV illumination, chemically reduce VOCs in the airflow to non-VOC molecules. discloses an air filtration unit comprising a plurality of baffles having air flow-through airflow spaces, and UV LEDs mounted on the baffles.

WO 2018/234633 A1, according to its abstract, states this publication discloses a filter unit connectable to a mobile communication device including a fan for generating an air flow inside the filter unit, electrodes covered with a photo catalytic material like TiO₂ in the air flow, UV-LEDs illuminating the electrodes, and outlet for the air flow directed in direction of user of filter unit.

GB 2574477 A, according to its abstract, states an air purifier comprises an elongate housing comprising an air inlet and an air outlet, wherein the elongate housing has a longitudinal axis. The air purifier also includes a photocatalytic reactor and a fan configured to direct air through the elongate housing from the air inlet to the air outlet via the photocatalytic reactor in an air flow direction. The air purifier also comprises a UV light source configured to direct UV light onto the photocatalytic reactor. The photocatalytic reactor is slanted to deflect, obliquely to the longitudinal axis air flowing in the air flow direction from the air inlet to the air outlet. Further inventions are disclosed relating to a photocatalytic air purifier characterised by a first air quality sensor, a second air quality and a processor; a photocatalytic air purifier characterised by a first air outlet and a second air outlet; an air purification system characterised by a photocatalytic air purifier and a remote control comprising an air quality sensor and a wireless interface for communicating with the air purifier, and the air purifier comprises a wireless communications interface; a remote control for an air purifier and a computer-implemented air quality monitoring system and a method of monitoring air quality. discloses an air purifier having a photocatalytic reactor and a UV light source.

GB 2472509 A, according to its abstract, states an air cleaner comprises an air moving unit (fan) creating an airflow through a channel, a photo-catalytic oxidation (PCO) element disposed in the airflow and an ultraviolet light source used to illuminate the PCO element. The light source may be Ultraviolet A (UV-A) Light Emitting Diode (LED) emitting UV light at wavelengths from 320 - 400 nanometres (nm). The PCO element may be titanium dioxide causing decomposition of water vapour in the airflow. A filter may be provided supporting the PCO element on a first face and a decomposing element on a second face. The decomposing element may be an ozone and/or volatile organic compound (VOC) decomposition element and removes odours. A particulate pre-filter may be provided. The UV LED catalyzes a reaction on the PCO element to clean the VOC decomposing element. The PCO can be a substrate comprising: a first coating comprising a VOC decomposing catalyst; a second coating comprising an ozone decomposing catalyst; and a third coating comprising titanium dioxide. An electrostatic precipitator may be disposed upstream of the PCO element.CN 209330845 U, according to its abstract, states the utility model provides a degradation device for source gas dirt of a livestock and poultry house. Including a chamber, a photocatalyst device is arranged in the cavity; the cavity is provided with an air inlet channel and an air outlet channel. The air inlet channel is provided with a filter screen, the exhaust channel is provided with a fan, the photocatalyst device comprises at least two baffles arranged in the cavity at intervals, a photocatalyst net and an ultraviolet lamp are arranged between every two adjacent baffles, the adjacent baffles are arranged on the two sides of the inner wall of the cavity in a staggered mode, and the baffles and the wall of the cavity jointly form a one-way serpentine channel. On one hand, most dust is prevented from entering the degradation device through the filter screen in the air inlet channel; on the other hand, remaining dust is further deposited through baffles arranged on the two sides of the inner wall of the cavity in a staggered mode; one part of remaining dust impacts the lower surface of the baffle along with airflow flowing to be adsorbed, and the other part of dust freely deposits on the upper surface of the baffle, so that a photocatalyst device in the degradation device can be prevented from being blocked, and the degradation device is suitable for degrading source gas dirt of livestock and poultry houses.

### SUMMARY

An air filtration unit as recited in claim 1 and a method for filtering air in an enclosed environment as recited in claim 11 are provided.

The following presents a simplified summary of the disclosure to provide a basic understanding to those of skill in the art. This summary is not an extensive overview of the disclosure and is not intended to identify key/critical elements of aspects of the disclosure or to delineate the scope of the disclosure. The sole purpose of this summary is to present some concepts disclosed herein in a simplified form as a prelude to the more detailed description that is presented later.

According to present aspects, air filtration apparatuses, systems, and methods are disclosed that address previous problems relating to the need to remove different types of contaminants from air contained within an enclosed or substantially enclosed environment. According to present aspects, apparatuses include, in synergistic combination, filtering and contaminant eliminating capabilities to simultaneous purify ambient air in an enclosed or substantially enclosed environment. More particularly, the presently disclosed apparatuses, systems, and methods filter or otherwise remove, in combination, from ambient air in an enclosed environment non-volatile organic compound particulates, volatile organic compounds (VOCs), and nitrous oxide-containing compounds.

According to the present disclosure, a "substantially enclosed environment" can include rooms and buildings with doors that are periodically opened, underground garages and surface garages that can retain air impurities for periods of time although entrances and exits may remain open for extended periods of time, hallways, meeting areas, meeting rooms, conference halls, as well as space stations, etc. As used herein, the term "enclosed environment" includes a "substantially enclosed environment".

According to one or more aspects of the present disclosure described and claimed herein, an air filtration unit enables the removal of particulates, including accumulated tars and other compounds resulting from, for example, smoked tobacco products, e-cigarettes, etc.; VOCs; and nitrous oxide-containing compounds from air in an enclosed environment, including where air can be directed through a duct.

According to present aspects, transverse to a longitudinal axis of an air ducts, an air filtration unit incorporating a HEPA filter, a VOC removal unit, and a nitrous oxide-containing compound removal unit is provided that can be positioned within or proximate to an air duct in order, for example, to protect the lifespan of the photocatalytic oxidation (PCO) component in the VOC removal unit.

A further aspect discloses an air filtration unit including an air duct, with the air duct including an air inlet at an air duct first end and an air outlet at an air duct second end, a high efficiency particulate air filter oriented proximate to or otherwise incorporated in the air inlet, and an airflow controller in communication with the air inlet. The air filtration unit further includes a carbon dioxide sensor in communication with the airflow controller, a pressure sensor in communication with the airflow controller. The air filtration unit includes an integrated ultraviolet light reactor located downstream from the high efficiency particulate air (HEPA) filter. The ultraviolet light reactor includes a plurality of baffles, with each baffle having a plurality of airflow spaces allowing airflow therethrough, with the baffles disposed at spaced locations within the duct between the air inlet and air outlet, and with the baffles being generally transverse to the longitudinal axis. The air filtration unit further includes a plurality of ultraviolet light emitting diodes mounted on each baffle, a porous and permeable photocatalytic oxidation filter module disposed between each pair of baffles, positioned generally transverse to the longitudinal axis, such that air flows through the photocatalytic oxidation filter module, and wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds. The air filtration unit further includes a porous and permeable nitrous oxide-adsorbing filter that can be, for example, a nitrous oxide-containing compound adsorbing chamber comprising a nitrous oxide-containing compound adsorbent, with the adsorbent in the adsorbent filter configured into an adsorbent bed, stack, etc. The nitrous oxide-adsorbing filter is disposed downstream of the UV light reactor. The terms "air duct" and "duct" are equivalent terms that have the same meaning herein and are interchangeable.

In another aspect, the air duct comprises a longitudinal axis.

In a further aspect, the carbon dioxide sensor is in communication with a controller.

In another aspect, the carbon dioxide sensor is in communication with the air inlet.

In another aspect, the carbon dioxide sensor is in communication with at least one of the air inlet and the air outlet.

In another aspect, the pressure sensor is in communication with the air inlet.

In a further aspect, the pressure sensor is in communication with at least one of the air inlet and the air outlet.

In another aspect, the pressure sensor is in communication with the controller.

In another aspect, the porous and permeable nitrous oxide-adsorbing filter comprises a solid amine-containing adsorbent.

In another aspect, the porous and permeable nitrous oxide-adsorbing filter comprises a packed solid adsorbent, with the packed solid adsorbent comprising at least one of: a cellular monolith arrangement and a granular media arrangement.

In another aspect, the porous and permeable nitrous oxide-adsorbing filter comprises a packed solid adsorbent, with the packed solid adsorbent comprising at least one of; an amine-containing compound, a metal-organic containing compound, and a zeolite.

In a further aspect, the porous and permeable nitrous oxide-adsorbing filter is oriented proximate to the air outlet.

In another aspect, the air filtration unit is configured to be removable from the air duct for replacement.

In another aspect, the air filtration unit is configured to be replaceable.

In a further aspect, one or more components of the air filtration unit are configured to be individually removable from the air duct.

In another aspect, one or more of the high efficiency particulate air filter, the ultraviolet light reactor, the photocatalytic oxidation filter module, and the nitrous oxide-adsorbing filter can be configured to be integrated into the air filtration unit as discrete components that are configured to be removable and replaceable.

In a further aspect, the air filtration unit further comprises one or more heat sink, wherein the one or more heat sink is configured to be disposed within the duct, and with the one or more heat sink further adapted to conduct heat away from the ultraviolet light emitting diodes.

In another aspect, the plurality of ultraviolet light emitting diodes are disposed both around a periphery of each baffle and between the airflow spaces, with the plurality of ultraviolet light emitting diodes configured to maximize ultraviolet illumination of an adjacent photocatalytic oxidation filter module.

In another aspect, the plurality of ultraviolet light emitting diodes are mounted on interior sides of baffles adjacent the air inlet and outlet, and the plurality of ultraviolet light emitting diodes are mounted on both sides of baffles, with the plurality of ultraviolet light emitting diodes configured to illuminate each photocatalytic oxidation filter module from both sides.

In another aspect, the photocatalytic oxidation filter module is spaced apart from the baffles such that both surfaces of each photocatalytic oxidation filter module are illuminated by ultraviolet light.

In a further aspect, the photocatalytic oxidation filter module comprises a plurality of filters, with each filter including one or more of a coarse foam, a fine foam, a fused quartz filament felt, or combination thereof, and wherein each filter is loaded with a catalyst including one or more of pure titanium dioxide (TiO₂), TiO₂ doped with iron (Fe-TiO₂), TiO₂ doped with carbon (C-TiO₂), or combination thereof.

In another aspect, the photocatalytic oxidation filter module comprises a plurality of catalyst-loaded filters selected, arranged, and configured to maximize ultraviolet illumination of each filter and through a complete depth of each filter layer.

In another aspect, the ultraviolet light reactor comprises four baffles and three photocatalytic oxidation filter modules, and wherein the photocatalytic oxidation filter modules comprise, in order from air inlet to air outlet,
1) R25 - CTR - TA - R25;
2) CTR - TA - Q25 - R25 - R25; and
3) R25 - CTR - TA - R25, wherein
   R25 is a coarse foam loaded with pure TiO₂;
   Q25 is a fused quartz filament felt loaded with pure TiO₂;
   CTR is a coarse foam loaded with C-TiO₂ and
   TA is a fine foam loaded with pure TiO₂.

In another aspect, the ultraviolet light reactor comprises four baffles and three photocatalytic oxidation filter modules, and wherein the photocatalytic oxidation filter modules comprise, in order from air inlet to air outlet,
1) R25 - TA - FTR - CTR;
2) R25 - CTA - FTR - Q25 - R25; and
3) R25 - TA - FTR - CTR; wherein
   R25 is a coarse foam loaded with pure TiO₂;
   Q25 is a fused quartz filament felt loaded with pure TiO₂;
   CTR is a coarse foam loaded with C-TiO₂;
   TA is a fine foam loaded with pure TiO₂; and
   FTR is a coarse foam loaded with Fe-TiO₂.

Aspects of the present disclosure further disclose a method for filtering air in an enclosed environment, with the method including monitoring carbon dioxide concentration in an enclosed environment, initiating an air purification cycle, directing an airflow to an air inlet of an air filtering unit, with the air filtration unit including an air duct having a longitudinal axis, with the air duct comprising an air inlet at an air duct first end and an air outlet at an air duct second end, a high efficiency particulate air (HEPA) filter unit oriented proximate to the air inlet, an airflow controller in communication with the air inlet, a carbon dioxide sensor in communication with the airflow controller, and a pressure sensor in communication with the airflow controller. The air filtration unit further includes an ultraviolet light reactor, with the ultraviolet light reactor further including a plurality of baffles, with each of the plurality of baffles having a plurality of airflow spaces allowing airflow therethrough the plurality of baffles, and with the plurality of baffles disposed at spaced locations within the air duct between the air inlet and air outlet, and with the baffles being generally transverse to the longitudinal axis. The air filtration unit and the ultraviolet light reactor further includes a plurality of ultraviolet light emitting diodes mounted on each baffle, a porous and permeable photocatalytic oxidation filter module disposed between each pair of baffles, generally transverse to the longitudinal axis, such that air flows through the photocatalytic oxidation filter module, and a porous and permeable nitrous oxide-adsorbing filter disposed downstream of the UV light reactor, and wherein each photocatalytic oxidation filter module contains one or more catalysts comprising at least one of titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds in the airflow to non-volatile organic compounds. The method further includes removing an amount of particulate from the airflow upstream from the photocatalytic oxidation filter modules, illuminating the photocatalytic oxidation filter modules with ultraviolet light from ultraviolet light emitting diodes mounted on each baffle, chemically reducing volatile organic compounds in the airflow to non-volatile organic compounds, removing an amount of nitrous oxide-containing compounds from the airflow downstream from the photocatalytic oxidation filter modules where the nitrous oxide-containing compounds can be produced due to nitrogen compound reductions.

In another aspect, the carbon dioxide sensor is in communication with the air inlet.

In a further aspect, a method further comprises illuminating the photocatalytic oxidation filter modules with ultraviolet light comprises disposing the ultraviolet light emitting diodes both around a periphery of each baffle and between the airflow spaces, so as to maximize the ultraviolet illumination of adjacent photocatalytic oxidation filter modules.

In another aspect, a method further comprises disposing the ultraviolet light emitting diodes on the baffles comprises disposing the ultraviolet light emitting diodes on interior sides of baffles adjacent the air inlet and outlet, and on both sides of baffles, so as to illuminate each PCO filter module from both sides.

In another aspect, a method further comprises illuminating each photocatalytic oxidation filter module with ultraviolet light comprises spacing each photocatalytic oxidation filter module apart from the baffles such that an entirety of both surfaces of each photocatalytic oxidation filter module is illuminated by ultraviolet light.

In another aspect, the method further includes conducting heat from the ultraviolet light emitting diodes away from the ultraviolet light emitting diodes via one or more heats sinks, with the one or more heat sinks disposed within the air duct.

In a further aspect, the method further includes selecting and arranging a plurality of catalyst-loaded filters to form the photocatalytic oxidation filter module so as to maximize illumination of the plurality of catalyst-loaded filters.

### BRIEF DESCRIPTION OF THE DRAWINGS

The present disclosure will now be described more fully hereinafter with reference to the accompanying drawings, in which aspects of the disclosure are shown. However, this disclosure should not be construed as limited to the aspects set forth herein. Rather, these aspects are provided so that this disclosure will be thorough and complete, and will fully convey the scope of the disclosure to those skilled in the art. Like numbers refer to like elements throughout.
Figure 1 is a perspective view of an example aircraft air filtration and VOC removal unit.
Figure 2 is a plan view of the example VOC removal unit, showing dimensions according to one aspect.
Figure 3 is a cross-section view of the example VOC removal unit, showing dimensions of the inlet/outlet and duct according to one aspect.
Figure 4 is a side view of an example baffle.
Figure 5 is a section view of an exemplary VOC removal unit showing the spatial relationship between baffles, UV LEDs, and filter modules, according to present aspects.
Figure 6 is a perspective view of an exemplary VOC and nitrous oxide-containing particle removal unit showing the spatial relationship between baffles, UV LEDs, and filter modules, according to present aspects.
Figure 7 is an end view of an air inlet section of an exemplary air filtration unit.
Figure 8 is an end view of an air outlet section of an exemplary air filtration unit.
Figure 9 is a flow diagram of an exemplary method of air filtration employing present apparatuses and systems.
Figure 10 is a flow diagram of an exemplary method of air filtration employing present apparatuses and systems.
Figure 11 is a flow diagram of an exemplary method of air filtration employing present apparatuses and systems.
Figure 12 is a flow diagram of an exemplary method of air filtration employing present apparatuses and systems.

### DETAILED DESCRIPTION

For simplicity and illustrative purposes, the present disclosure is described by referring mainly to an exemplary aspect thereof. In the following description, numerous specific details are set forth to provide a thorough understanding of the present disclosure. However, it will be readily apparent to one of ordinary skill in the art that the aspects of the present disclosure can be practiced without limitation to these specific details. In this description, well known methods and structures have not been described in detail so as not to unnecessarily obscure the present disclosure.

Present aspects are directed to apparatuses, systems, and methods for VOC removal from air in enclosed environments that can considered to be large, enclosed environments including, for example, terrestrial environments such as, for example, rooms, transportation terminals, smoke rooms, hallways, meeting areas, meeting rooms, conference halls, as well as non-terrestrial environments including, for example, extra-terrestrial rooms, buildings, space stations, etc. In addition, further present aspects are directed to apparatuses, systems, and methods for protected VOC and nitrogen-containing compound removal from the enclosed environments disclosed herein.

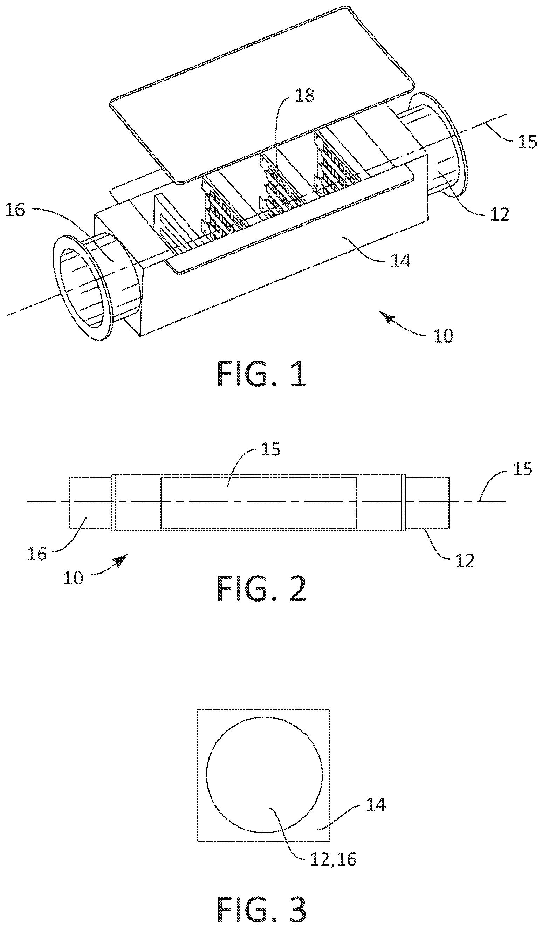

Figure 1 depicts an example of an air filtration and VOC removal unit 10 (hereinafter referred to as simply a "VOC removal unit" 10), according to one aspect of the present disclosure. The VOC removal unit 10 operates to remove VOCs from air by photocatalytic oxidation (PCO), as will be explained in greater detail herein. The design maximizes airflow through the VOC removal unit 10 and minimizes heat generation and a thermal gradient across it, consistent with the maximum achievable UV illumination of PCO filters. As used herein, with respect to the present aspects, the terms "photocatalytic oxidation filter module" and "porous and permeable photocatalytic oxidation filter module" are equivalent terms and are used interchangeably.

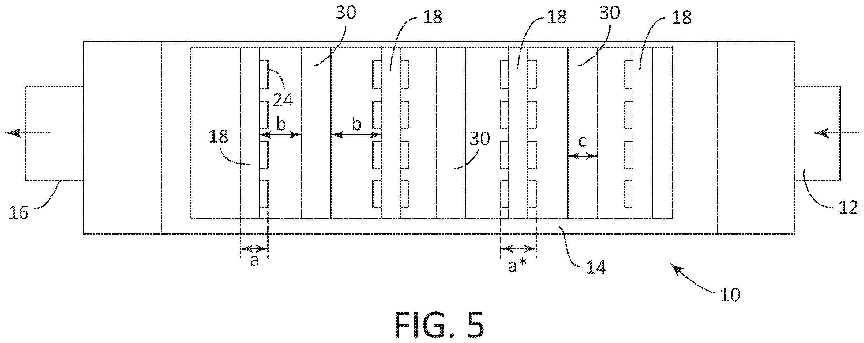

Figure 1 is a perspective view of an example VOC removal unit 10, with one side removed to reveal internal components. Figures 2 and 3 are sectional views of the VOC removal unit 10 according to one aspect, and Figure 5 is a section view showing the spacing and relationship of various components.

The VOC removal unit 10 comprises an air inlet 12, a duct 14 having a longitudinal axis 15, and an air outlet 16. In the representative aspect of the VOC removal unit 10 depicted in the figures, the air inlet 12 and outlet 16 have a circular cross-sectional shape, and the duct 14 has a square cross-section. However, those of skill in the art will readily recognize that other shapes can be utilized, within the scope of the present disclosure.

Figure 5 depicts the overall operative structure of the VOC removal unit 10 incorporated into the present air filtration unit. A plurality of baffles 18 is disposed at spaced locations within the duct 14. Between each pair of baffles 18, a PCO filter module 30 comprises a plurality of filters, each of which is loaded with a photoactive catalyst. The number and spacing (as explained further below) of baffles 18 and PCO filter modules 30 is representative. In other aspects, more or fewer of each may be provided. In general, the number of baffles 18 will always exceed the number of PCO filter modules 30 by one; with baffles 18 disposed at both ends and in between each pair of PCO filter modules 30. Ultraviolet (UV) light emitting diodes (LED) 24 mounted on the baffles 18 illuminate both sides of the filter modules 30 with UV light to maximize illumination of photocatalytic coatings on material in the filter modules 30. Since the photocatalytic coatings require UV light as a catalyst to convert VOCs to non-VOC molecules, maximizing the illumination of PCO modules 30 with UV light maximizes the effectiveness of the VOC removal unit 10. Cabin air directed through the VOC removal unit 10 passes through the baffles and PCO filter modules 30. As explained further herein, UV light illuminating the PCO filter modules 30 photoactivates catalysts loaded therein, initiating a chemical photocatalytic oxidation process that reduces VOCs in the air to non-VOCs molecules, such as carbon dioxide and water.

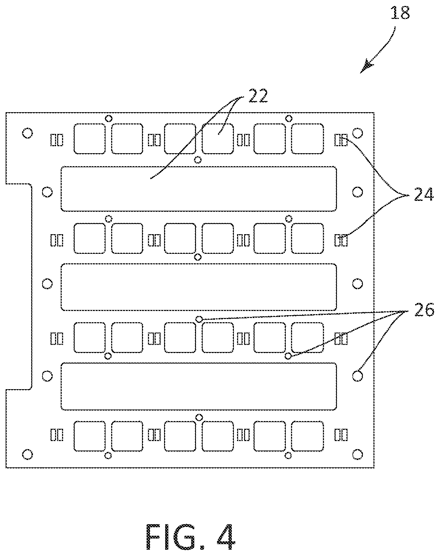

The baffles 18 are disposed at spaced locations within the duct 14, between the air inlet 12 and air outlet 16. The baffles 18 are disposed generally transverse to the longitudinal axis 15 of the duct 14. As depicted in Figure 4, each baffle 18 has a plurality of airflow spaces 22 formed in it, allowing airflow therethrough. A plurality of UV LEDs 24 is mounted on each baffle. The UV LEDs 24 are disposed both around the periphery of each baffle 18, and between the airflow spaces 22, so as to maximize the UV illumination of adjacent filter modules 30. The UV LEDs 24 are mounted on the interior sides of baffles 18 adjacent the air inlet 12 and outlet 16 and are mounted on both sides of all other baffles 18, so as to illuminate the filter modules 30 from both sides. Mounting UV LEDs 24 on all baffles 18 ensures maximum illumination of all filter modules 30, for maximum photocatalytic effect.

The efficacy of the VOC removal unit 10 is greatest when the UV LEDs 24 are operated at high power (~500 mA), thus generating a large luminous flux of UV light to activate the photoactive catalysts in the filter modules 30. However, this can generate heat, which warms the air flowing through the duct 14, increasing the load on aircraft air conditioning equipment. In one aspect a heat sink is connected to at least one, and preferably to each baffle 18 that includes LEDs 24, using heat sink mounting holes 26. This is done to maintain the life of the UV-LED lights by maintaining lower temperatures on their surface in low flow conditions, prolonging their life. In certain filter configurations (designed for those with higher flow rates) the heat sinks may not be necessary.

A porous and permeable PCO filter module 30, comprising a plurality or "stack" of filters, is disposed between each baffle 18. The PCO filter modules 30 are disposed generally transverse to the longitudinal axis 15 of the duct 14, such that air flows through the PCO filter module 30. Each PCO filter module 30 contains one or more catalysts which, when illuminated by UV light, are operative to chemically reduce VOCs to non-VOC molecules. Maximum UV illumination of all filters in each filter module 30 is thus desired, to maximize the efficacy of VOC removal. Accordingly, the PCO filter modules 30 are spaced apart from the baffles such that the entirety of both surfaces of each PCO filter module is illuminated by UV light.

If a PCO filter module 30 were directly adjacent a baffle 18, only spots on the surface of the PCO filter module 30 that contact a UV LED 24 would be illuminated. As the spacing between the PCO filter module 30 and the baffle 18 increases, the illumination spot sizes increase, and the photonic efficiency decreases. The optimal spacing is that distance at which the illumination spots just overlap, fully illuminating the entire facing surface of the PCO filter module 30, increasing the spacing beyond this distance reduces the luminous flux of UV light. In the aspect depicted in Figure 5, the distance between each face of a filter module 30 and the facing baffle 18 is 20mm (dimension "b"); at this distance, 500mA of power applied to the UV LEDs 24 yields a light intensity of over 20mW/cm². The filter modules 30 in this aspect are 40mm thick (dimension "c"). In the aspect depicted, the baffles 18, measured to the outermost protruding UV LEDs 24, are 15mm thick for those adjacent to the air inlet 12 and outlet 16, which have UV LEDs 24 mounted on only the interior-facing sides (dimension "a"), and 30mm thick for interior baffles 18, which have UV LEDs 24 mounted on both sides (dimension "a*"). These dimensions are exemplary. Present aspects contemplate alternate relative spacing of components for VOC removal units of different size or shape.

The filters in each PCO filter module 30 are loaded with some form of titanium dioxide (TiO₂). Photocatalytic oxidation occurs in the VOC removal unit 10 by illuminating the TiO₂ in the filters with UV light, generating hydroxyl radicals (OH•) by reaction with water molecules in the air. The free radicals, in turn, oxidize VOCs into non-VOC molecules - primarily carbon dioxide (CO₂) and water (H₂O). These are returned to the airflow, avoiding the accumulation of contaminants.

Titanium dioxide is a light-sensitive semiconductor, which adsorbs electromagnetic radiation in the near UV region. The most common natural form of TiO₂ is the mineral rutile. Other forms of TiO₂ are anatase (also known as octahedrite) and brookite (an orthorhombic mineral). TiO₂, when used as a photoactive catalyst, is primarily anatase, with a small amount of rutile. The anatase form of TiO₂ requires higher light energy than the rutile form and shows a stronger photoactivity. The energy difference between the valence and the conductivity bands of a TiO₂ molecule in the solid state is 3.05eV for rutile and 3.29eV for anatase, corresponding to a photonic absorption band at <415 nm for rutile and <385 nm for anatase.

Absorption of light energy causes an electron to be promoted from the valence band to the conduction band. This electron, and the simultaneously created positive "electron hole," can move on the surface of the solid, where it can take part in redox reactions. In particular, water molecules in vapor state in the air are adsorbed onto the TiO₂ surface where they react with the free electron, generating hydroxyl radicals (OH•). These radicals are uncharged, short-lived, highly reactive forms of hydroxide ions (OH-), bearing considerable oxidizing power. The OH• radicals can cause complete oxidation of organic compounds to carbon dioxide and water. In some aspects, the OH• radicals reduce VOCs to the following end products:
organic molecules → CO₂ + H₂O
organic N-compounds → HNO₃ + CO₂ + H₂O
organic S-compounds → H₂SO₄ + CO₂ + H₂O
organic Cl-compounds → HCl + CO₂ + H₂O.
Although the primary application of photocatalytic oxidation in the VOC removal unit 10 is to reduce VOCs into non-VOC molecules, the process also kills contaminants in bioaerosols, such as bacteria, molds, and fungus. In general, reduction of VOC levels in cabin air enhances comfort of passengers.

The photoactivity of TiO₂ is known and has commercial applications. AEROXIDE^{®} P25 is a nanostructured, fine-particulate pure titanium dioxide with high specific surface area. The product, available from Evonik Industries of Parsippany, NJ. (AEROXIDE^{®} P25), is a fine white powder with hydrophilic character caused by hydroxyl groups on the surface. It consists of aggregated primary particles. The aggregates are several hundred nm in size and the primary particles have a mean diameter of approximately 21nm. The Brunner-Emmett-Teller (BET) theory can be used to measure the surface area of the solid or porous material selected, optionally in conjunction with transmission electron microscopy (TEM) imaging to confirm pore size. Further, pore size distribution can be evaluated by Barrett-Joyner-Halenda (BJH) interpretation. The weight ratio of anatase to rutile is approximately 80/20. AEROXIDE^{®} P25 is sold commercially as a photoactive catalyst. With its high purity, high specific surface area, and combination of anatase and rutile crystal structure, AEROXIDE^{®} P25 is widely used for catalytic and photocatalytic applications. Other forms of pure TiO₂ may also be used in PCO filter modules 30 in the VOC removal unit 10.

Additionally, the inventors have found that doping TiO₂ with iron (Fe-TiO₂) and carbon (C-TiO₂) yield superior photocatalytic results. The UV-PCO reactor relies on adsorption of the organic compounds onto the surface of the catalyst to enable breakdown of the compounds. Doping the TiO₂ with carbon or iron increases the sorption capacity of the catalyst, which allows for greater removal of VOCs from the airstream. Through doping with metal and non-metal agents the band gap energy level of TiO₂ is lowered and electron-hole pair mechanism is kept constant with a longer duration for higher light absorption capability which results in better efficiency.

Table 1 below lists pre- and post-filtering concentrations of various representative VOCs (i.e., ethanol, or EtOH; acetone; and limonene) for pure TiO₂, Fe-TiO₂, and C-TiO₂, when loaded onto various filter media types. It is clear from these data that Fe-TiO₂, and C-TiO₂ provide superior VOC removal results, as compared to pure TiO₂.

**Table 1 - Relative Performance of Photoactive Catalysts**

| Substrate | VOC Type | Initial VOC Concentration (ppb) | | Final VOC Concentration (ppb) | | Efficiency |
|---|---|---|---|---|---|---|
| pure TiO₂ | | | | | | |
| Coarse Foam | EtOH | | 423.40 | | 350.22 | 17.2% |
| | Acetone | | 117.30 | | 78.80 | 32.8% |
| Fine Foam | EtOH | NA | | NA | | NA |
| | Acetone | NA | | NA | | NA |
| Coarse/Fine Foam | EtOH | | 449.00 | | 320.20 | 28.6% |
| | Acetone | NA | | NA | | NA |

| Fe-doped TiO₂ | | | | | | |
|---|---|---|---|---|---|---|
| Coarse Foam | EtOH | | 389.20 | | 198.70 | 48.9% |
| | Acetone | | 74.50 | | 30.30 | 59.3% |
| Fine Foam | EtOH | NA | | NA | | NA |
| | Acetone | NA | | NA | | NA |
| Coarse/Fine Foam | EtOH | | 499.30 | | 196.70 | 60.6% |
| | Acetone | NA | | NA | | NA |
| | Limonene | | 39.30 | | 9.30 | 68.7% |

| C-doped TiO₂ | | | | | | |
|---|---|---|---|---|---|---|
| Coarse Foam | EtOH | NA | | NA | | NA |
| | Acetone | NA | | NA | | NA |
| Fine Foam | EtOH | NA | | NA | | NA |
| | Acetone | NA | | NA | | NA |
| Coarse/Fine | EtOH | | 417.40 | | 203.45 | 51.2% |
| Foam | Acetone | | 152.30 | | 80.90 | 46.8% |

| | | | | | | |
|---|---|---|---|---|---|---|
| NA: Not | | | | | | |

### Available

The photoactive catalyst - whether AEROXIDE^{®} P25 (considered to be a "pure TiO₂" according to the present disclosure), other pure TiO₂, Fe-TiO₂, or C-TiO₂ - is loaded into a porous and air- and light-permeable filter. In one aspect, the photoactive catalyst is adhered to all surface area of the filter, including within pores and passages running throughout the volume of the filter medium. In at least one aspect, the catalyst is deposited by a dip coating method, followed by drying at 80 -100 °C. Other methods of catalyst deposition may be used.

The type of substrate and coating methods have important effects on coating stability, photocatalytic, and mechanical performance of the filters. Porous metal substrates offer better toughness, better malleability, and lower cost than ceramic substrates. However, using metal substrate generally results in peeling coatings with cracks. This occurs at heating stage and due to difference in thermal expansion coefficients between the TiO₂ and the substrate metal.

Porous TiO₂ filters are commonly employed to avoid this problem. Such filters are commonly prepared by coating a TiO₂ sol, slurry, or precursor liquid onto ceramic substrates, metal meshes or ceramic or metallic foam by different coating methods. After coating application, heat treatment necessary for photocatalysts activity around 500 °C is generally conducted.

In one aspect, three filter types are used in PCO filter modules 30: coarse foam, fine foam, and fused quartz filament felt. Both the porosity (number and size of pores, or voids) and the permeability (ability of fluid to flow through, which is related to interconnectivity of the pores) of each type of filter type are selected based on a contemplated use relative to VOCs of interest. The Brunner-Emmett-Teller (BET) theory can be used to measure the surface area of the solid or porous material selected, optionally in conjunction with transmission electron microscopy (TEM) imaging to confirm pore size. Further, pore size distribution can be evaluated by Barrett-Joyner-Halenda (BJH) interpretation. Porosity contributes to the surface area for adhering more photocatalytic coatings. Permeability impacts the selected volume of air that can flow through the PCO filter modules 30 to remove VOCs from the cabin air of a large aircraft, for example.

The coarse foam is a relatively open foam, with average pores size of approximately 2540um, and high permeability. The coarse foam filter is approximately 10mm thick. A suitable coarse foam is available from Recemat BV of the Netherlands. This material can be uniformly coated with catalyst. The coarse foam filter allows much of the incident UV light to penetrate the foam, thus illuminating successive filters. For this reason, in some aspects, a coarse foam filter is at both exterior positions of a "stack" of filters forming a PCO filter module 30. The position of coarse foam filter is also maintained on the outside of whole filter stack to start the VOC degradation in a lower specific surface area to very high specific surface area in fine foam filters.

The fine foam is a denser foam, with average pores size of approximately 800um, and lower permeability as compared to coarse foam but with a higher surface area. The Brunner-Emmett-Teller (BET) theory can be used to measure the surface area of the solid or porous material selected, optionally in conjunction with transmission electron microscopy (TEM) imaging to confirm pore size. Further, pore size distribution can be evaluated by Barrett-Joyner-Halenda (BJH) interpretation. Accordingly, the fine foam filter is thinner than the coarse foam filter, at approximately 2-4mm, to maintain robust airflow. This material is more difficult to coat uniformly with catalyst. A suitable fine foam is available from Alantum GMBH of Germany.

Another type of material is made from fused quartz filaments. A suitable felt of this type is QUARTZEL^{®} felt, available from Magento of the USA. The QUARTZEL^{®} felt is difficult to coat uniformly and presents a high resistance to air flow. Accordingly, it is used sparingly. In one aspect, only one of three PCO filter modules 30 in a VOC removal unit 10 include a fused quartz filament filter, and that module 30 includes only a single such filter.
Each PCO filter module 30 comprises a plurality of catalyst-loaded filters selected and arranged to maximize UV illumination of all of the filters. That is, according to present aspects, in the present air filtration unit, the photocatalytic oxidation filter module comprises a plurality of catalyst-loaded filters selected and arranged to maximize ultraviolet illumination of each filter and through a complete depth of each filter layer.

With, in some aspects, three filter media and four types of photoactive catalysts, there are a dozen combinations of PCO filters from which to select. The number of permutations of which of these filters to stack into a filter module 30, in which order, is very large. Furthermore, different PCO filter modules. That is, different selections and arrangements of photoactive-catalyst-loaded filters can be placed in different locations along the duct 14 of the VOC removal unit 10.

In one aspect, as depicted in Figure 5, an air filtration and volatile organic compound (VOC) removal unit 10 includes an air duct 14 having a longitudinal axis 15, an air inlet 12 at one end, and air outlet 16 at the other end; a plurality of baffles 18 (Figure 4), each having a plurality of open spaces 22 allowing airflow therethrough, disposed at spaced locations within the duct 14 between the air inlet 12 and air outlet 16, the baffles 18 being generally transverse to the longitudinal axis 15; a plurality of ultraviolet (UV) light emitting diodes (LED) 24 mounted on each baffle 18; and a porous and permeable photocatalytic oxidation (PCO) filter module 30 disposed between each pair of baffles 18, generally transverse to the longitudinal axis 15, such that air flows through the PCO filter module 30. Each PCO filter module 30 contains one or more catalysts comprising titanium dioxide (TiO₂), TiO₂ doped with iron (Fe-TiO₂), TiO₂ doped with carbon (C-TiO₂), or combinations thereof, which, when illuminated by UV light, are operative to chemically reduce VOCs to non-VOC molecules. This provides a compact, lightweight, low-power means for removing VOCs.

In one aspect, the VOC removal unit further includes one or more heat sinks disposed within the duct and adapted to conduct heat away from the UV LEDs away and maintain their lifespan. This prevents overheating of the LEDs to prolong their life in a low flow situation.

In one aspect, the UV LEDs are disposed both around a periphery of each baffle and between the airflow spaces so as to maximize the UV illumination of adjacent PCO filter modules. The UV LEDs are mounted on interior sides of baffles adjacent the air inlet and outlet and are mounted on both sides of all other baffles, so as to illuminate the PCO filter modules from both sides. The PCO filter modules are spaced apart from the baffles such that the entirety of both surfaces of each PCO filter module is illuminated by UV light. These features ensure maximum and even illumination of the PCO filter modules by UV light.

Each PCO filter module comprises a plurality of filters, each filter selected from the group consisting of a coarse foam, a fine foam, and a fused quartz filament felt, and each filter is loaded with a catalyst which may be one or more of pure titanium dioxide (TiO₂), TiO₂ doped with iron (Fe-TiO₂), and TiO₂ doped with carbon (C-TiO₂), and combinations thereof. In one aspect, each PCO filter module comprises a plurality of catalyst-loaded filters selected and arranged to maximize UV illumination of all of the filters. These materials have high durability and the arrangements facilitate the removal of VOCs.

The VOC removal unit 10 comprises four baffles 18 and three PCO filter modules 30. The PCO filter modules 30 comprise, in order from air inlet 12 to air outlet 16,
1) R25 - CTR - TA - R25;
2) CTR - TA - Q25 - R25 - R25; and
3) R25 - CTR - TA - R25; wherein
   R25 is a coarse foam loaded with pure TiO₂;
   Q25 is a fused quartz filament felt loaded with pure TiO₂;
   CTR is a coarse foam loaded with C-TiO₂ and
   TA is a fine foam loaded with pure TiO₂.

In another aspect, with the same numbers of baffles 18 and PCO filter modules 30, the PCO filter modules 30 comprise, in order from air inlet 12 to air outlet 16,
1) R25 - TA - FTR - CTR;
2) R25 - CTA - FTR - Q25 - R25; and
3) R25 - TA - FTR - CTR; wherein
   FTR is a coarse foam loaded with Fe-TiO₂.

Based on the information disclosed herein, those of skill in the art may devise numerous other selections and arrangements of both photoactive catalyst-loaded filters in each PCO filter module 30, and the PCO filter modules 30 in the VOC removal unit 10, within the scope of the present disclosure.

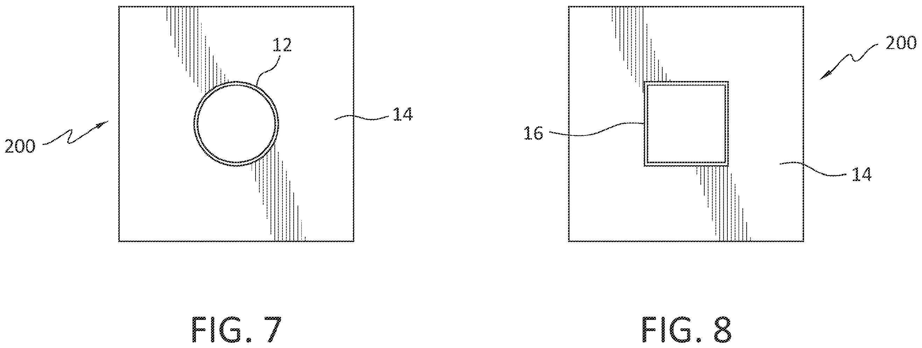

According to further aspects, Figure 7 shows an exemplary air purification system and apparatus that can implement the PCO filter modules of the types shown, for example, in Figure 5, in combination with presently disclosed selected additional filter system components integrated into the system to further enhance particulate removal (including non-VOC particulate removal) upstream from the PCO filter modules, and further effect nitrous oxide-containing compound removal from an airflow downstream from the PCO filter modules. According to present aspects, placement of an HEPA filter upstream of the UV-PCO portion of the present air filtration unit removes tar/particles from the airflow directed into the present air filtration unit that may otherwise impact the surfaces of the PCO, and that could substantially reduce the efficiency of the ultraviolet light reactor(s).

According to present aspects, nitrous oxide (NO₂, NOₓ, NO) adsorbent such as, for example, an amine adsorbent, is oriented in the nitrous oxide-adsorbing filter downstream of the ultraviolet light reactor(s), as nitrous oxides are a byproduct of reactions between ammonia (a product of, for example, smoking), and oxygen in the presence of ultraviolet light. Likewise, nitrous oxides are also highly present within indoor enclosed environments including, for example, an airport, a bus terminal, a railway terminal, a planetary habitat, and other terrestrial transportation environments, etc. To further purify ambient air in such environments, present aspects reduce the exposure to nitrous oxide-containing compounds as the contemplated nitrous oxide removal region, including a nitrous oxide-adsorbing filter, in the presently disclosed air purification unit significantly reduces the nitrous oxide concentrations in the ambient air present in, for example, an occupied area of a terrestrial transportation environment including, for example, an airport, a smoking room in an airport, etc.

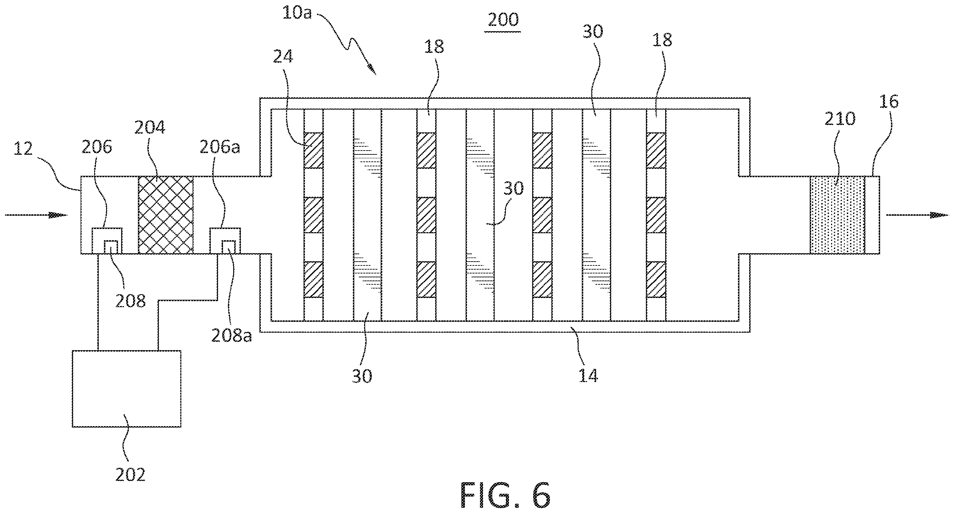

As shown in Figure 6, according to present aspects, an air filtration apparatus 200 incorporates a filter 204 that can be a high-efficiency particulate air HEPA filter located proximate to or otherwise incorporated into an air inlet 12 of a VOC removal unit 10a of the type shown in Figure 5 and described herein. According to present aspects, the HEPA filter is oriented upstream of the VOC removal unit 10a. HEPA filters are also known as high-efficiency particulate adsorbing and high-efficiency particulate arresting filters representing an efficiency standard of air filter. Filters meeting the HEPA standard must satisfy certain levels of efficiency. Common standards require that a HEPA air filter must remove from the air passing therethrough of at least 99.95% (European Standard) or 99.97% (ASME, U.S. D.O.E.) of particles having a diameter equal to 0.3 µm or greater, with the filtration efficiency increasing for particle diameters both less than and greater than 0.3 µm.

According to present aspects, HEPA filters can comprise a mat of ordered or randomly arranged fibers. The fibers can include fiberglass having diameters between 0.5 and 2.0 µm. The air space between HEPA filter fibers is typically much greater than 0.3 µm. Unlike sieves or membrane filters, where particles smaller than openings or pores can pass through, HEPA filters are designed to target a range of particle sizes. These particles are trapped (they stick to a fiber) through a combination of the mechanisms including diffusion, interception, and impaction. As used in accordance with the present aspects, and as shown in Figure 6, airflow introduced into the air filtration apparatuses and systems first encounters the HEPA filter that is located upstream from the VOC removal unit 10a for the purpose of removing particulates from the introduced airflow upstream of the VOC removal unit that incorporates the PCO filter modules (that, in turn, employ the UV photocatalysis) with the VOC removal unit being primarily responsible for the subsequent removal of VOCs from the airflow flowing through the unit.

According to present aspects, the disclosed apparatuses, systems, and methods can implement a variable speed or diverted air system based upon the CO₂ of the occupied space in an enclosed environment to minimize exposure of the air purifier to contaminants; having the potential to increase the lifespan of the technology and minimize maintenance costs.
When the NOx is primarily produced within the unit during operation, diverting an airflow can assist in preventing the exposure of the unit to a selected level of CO₂ that can be directed into the air filtration unit from the occupied space in, for example, an enclosed environment.

Figure 6 shows an airflow controller 202 that is in communication with a first and second carbon dioxide (CO₂) sensors 206, 206a, respectively, with the CO₂ sensors 206, 206a further comprising or otherwise in communication with first and second pressure sensors 208, 208a respectively incorporated into or proximate to the air inlet 12. Pressure sensors 208, 208a are in communication with airflow controller 202 with the pressure sensors 208, 208a configured to monitor and determine changes in system pressures, with the pressure sensors 208, 208a further able to monitor the performance of the HEPA filter, for example, to output information regarding to potential clogging of, for example, a HEPA filter.

As shown in Figure 6, the airflow controller can initiate, terminate, modify, and otherwise direct and control air in a surrounding environment to form an airflow into the air filtration apparatus 200. Air in a surrounding environment can include air that is, for example, within an enclosed environment, and the enclosed environment can be, for example, a room in a building, a terminal, a warehouse etc., and that can be an enclosed environment in or proximate to a terrestrial transportation environment such as, for example, an airport terminal, a railway terminal, a bus terminal, a raceway, etc.

While the presently disclosed particulate and VOCs removed from ambient air in a particular surrounding can accomplish a certain desired level of purification of ambient air, as shown in Figure 6, the air filtration unit 200 further comprises a nitrous oxide-adsorbing filter 210 that can be a porous and permeable nitrous oxide-adsorbing chamber further comprising, for example, a bed, stack etc. that is disposed downstream from the VOC removal unit 10a for the purpose of removing nitrous oxide-containing compounds from the air directed through the air filtration apparatus. The nitrous oxide-containing compounds that can be adsorbed by the nitrous oxide-adsorbing filter can include, for example, NO₂, NOₓ, NO. The nitrous oxide-adsorbing filter can be oriented within the air filtration unit at a point downstream from the VOC removal unit, and can be located proximate to the air outlet, or otherwise incorporated into the air outlet.

The nitrous oxide-adsorbing filter can include a solid form amine-containing packed bed, a cellular monolith, a granular media set-up, etc., and the nitrous oxide-adsorbing filter can have a separate frame housing the nitrous oxide-adsorbing filter, or the frame can be built into or otherwise incorporated and formed as a part of (e.g., integral with) the air filtration unit air outlet. The amine will be immobilized into a solid form (e.g., a monolithic contractor), rather than utilizing a liquid membrane. While amines and amine-containing compounds are contemplated for use in the nitrous oxide-adsorbing filter, other potential nitrous oxide adsorbents can include metal organic compounds, zeolites, etc., alone or in combination.

Figure 7 shows an end view of the air filtration unit 200 showing the air inlet 12 framed by duct 14. Figure 8 shows an end view of the air outlet end of a presently disclosed air filtration unit 200, with the air outlet 16 framed by the duct 14. While the air intake is shown in a circular configuration and the duct perimeter and air outlet are shown as substantially square, it is understood that presently disclosed filtration units can be configured into any geometry as desired and according to the constraints presented by ducts in ductwork, including ducts that can be pre-existing ducts into which the presently disclosed air filtration units can be retrofitted, for example.

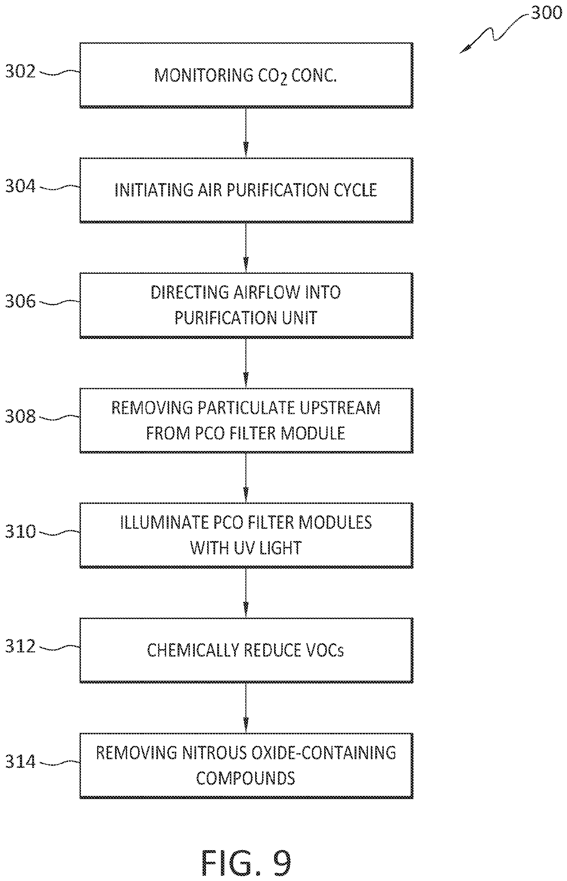

Figures 9, 10, 11, 12 are flowcharts showing exemplary methods implementing the apparatuses and systems shown at least in Figures 5, 6, 7, and 8.

As shown in Figure 9, a presently disclosed method is disclosed for filtering air in an enclosed environment, with the method 300 including monitoring 302 carbon dioxide concentration in an enclosed environment, initiating 304 an air purification cycle, directing 306 an airflow to an air inlet of an air filtration unit, with the air filtration unit including an air duct having a longitudinal axis, said air duct comprising an air inlet at a first end and an air outlet at a second end, a high efficiency particulate air (HEPA) filter unit oriented proximate to the air inlet, an airflow controller in communication with the air inlet, a carbon dioxide sensor in communication with the airflow controller, and a pressure sensor in communication with the airflow controller. The air filtration unit further includes an ultraviolet light reactor that further includes a plurality of baffles, with each baffle having a plurality of airflow spaces allowing airflow therethrough, disposed at spaced locations within the duct between the air inlet and air outlet, and with the baffles being generally transverse to the longitudinal axis. The air filtration unit further includes a plurality of ultraviolet light emitting diodes mounted on each baffle, a porous and permeable photocatalytic oxidation filter module disposed between each pair of baffles, generally transverse to the longitudinal axis, such that air flows through the photocatalytic oxidation filter module, and a porous and permeable nitrous oxide-adsorbing filter disposed downstream of the UV light reactor, and wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds. The method further includes removing 308 an amount of particulate from the airflow upstream from the photocatalytic oxidation filter modules, illuminating 310 the photocatalytic oxidation filter modules with ultraviolet light from ultraviolet light emitting diodes mounted on each baffle, chemically reducing 312 volatile organic compounds in the airflow to non-volatile organic compounds (thus removing VOCs from the airflow), and removing 314 an amount of nitrous oxide-containing compounds from the airflow downstream from the photocatalytic oxidation filter modules and wherein each photocatalytic oxidation filter module contains one or more catalysts comprising titanium dioxide (TiO₂), TiO₂ doped with iron (Fe-TiO₂), or TiO₂ doped with carbon (C-TiO₂) which, when illuminated by UV light, are operative to chemically reduce volatile organic compounds in the airflow to non-volatile organic compounds.

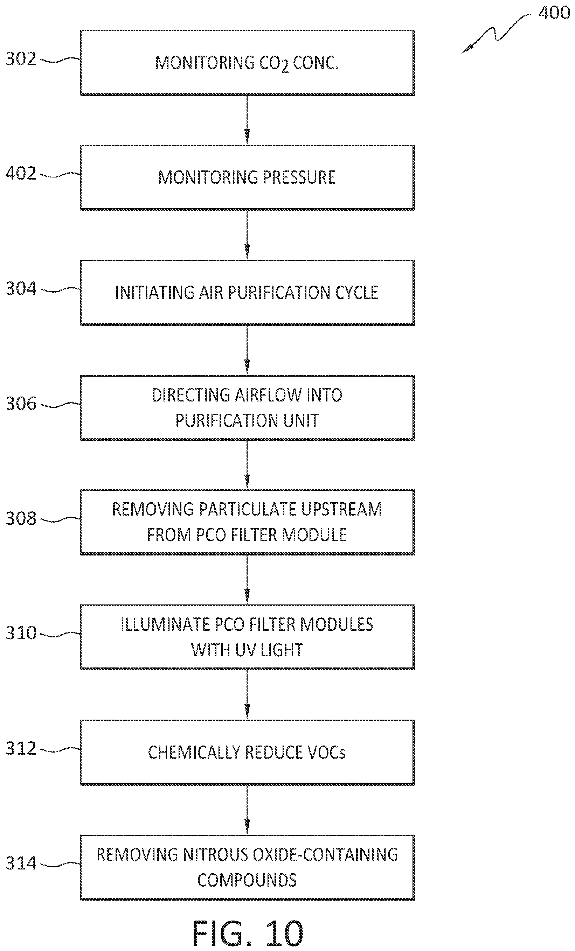

Figure 10 is a flowchart outlining a presently disclosed method for filtering air in an enclosed environment, with the method 400 including monitoring 302 carbon dioxide concentration in an enclosed environment and monitoring 402 pressure of the system for the purpose of sensing a pressure drop indicating a need for adjusting a system airflow capacity, for example. The pressure can be monitored at an air intake or an air outlet, with the pressure monitored by a sensor that can send a signal to a controller that can adjust airflow capacity through the unit and system, etc.

Method 400 shown in Figure 10 further shows initiating 304 an air purification cycle, directing 306 an airflow to an air inlet of an air filtration unit, with the air filtration unit including an air duct having a longitudinal axis, said air duct comprising an air inlet at a first end and an air outlet at a second end, a high efficiency particulate air (HEPA) filter unit oriented proximate to the air inlet, an airflow controller in communication with the air inlet, a carbon dioxide sensor in communication with the airflow controller, and a pressure sensor in communication with the airflow controller. The air filtration unit further includes an ultraviolet light reactor that further includes a plurality of baffles, with each baffle having a plurality of airflow spaces allowing airflow therethrough, disposed at spaced locations within the duct between the air inlet and air outlet, and with the baffles being generally transverse to the longitudinal axis. The air filtration unit further includes a plurality of ultraviolet light emitting diodes mounted on each baffle, a porous and permeable photocatalytic oxidation filter module disposed between each pair of baffles, generally transverse to the longitudinal axis, such that air flows through the photocatalytic oxidation filter module, and a porous and permeable nitrous oxide-adsorbing filter disposed downstream of the UV light reactor, and wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds. The method further includes removing 308 an amount of particulate from the airflow upstream from the photocatalytic oxidation filter modules, illuminating 310 the photocatalytic oxidation filter modules with ultraviolet light from ultraviolet light emitting diodes mounted on each baffle, chemically reducing 312 volatile organic compounds in the airflow to non-volatile organic compounds (thus removing VOCs from the airflow), and removing 314 an amount of nitrous oxide-containing compounds from the airflow downstream from the photocatalytic oxidation filter modules and wherein each photocatalytic oxidation filter module contains one or more catalysts comprising titanium dioxide (TiO₂), TiO₂ doped with iron (Fe-TiO₂), or TiO₂ doped with carbon (C-TiO₂) which, when illuminated by UV light, are operative to chemically reduce volatile organic compounds in the airflow to non-volatile organic compounds.

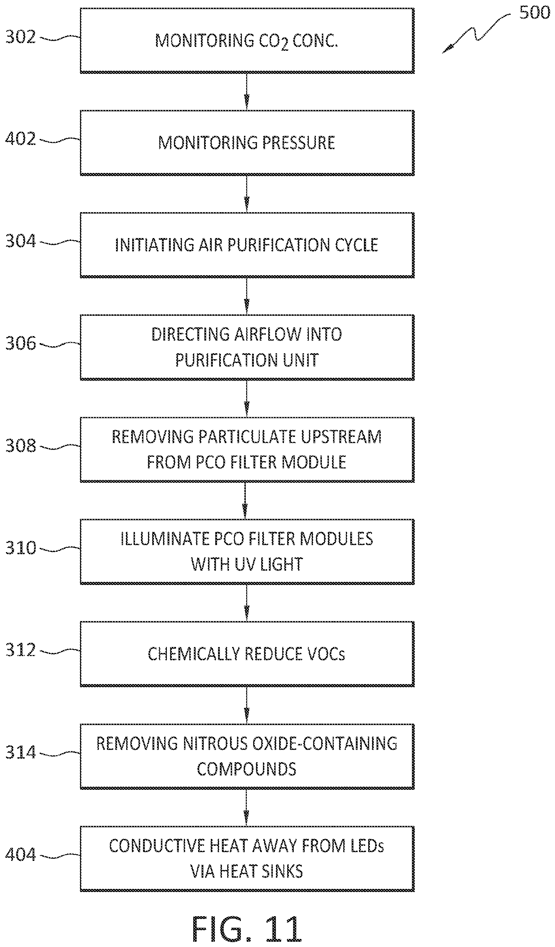

Figure 11 is a flowchart outlining a presently disclosed method for filtering air in an enclosed environment, with the method 500 including monitoring 302 carbon dioxide concentration in an enclosed environment, monitoring 402 pressure of the system for the purpose of sensing a pressure drop indicating that the need for adjusting a system airflow capacity, for example. The pressure can be monitored at an air intake or an air outlet, with the pressure monitored by a sensor that can send a signal to a controller that can adjust airflow capacity through the unit and system, etc.

Method 500 shown in Figure 11 further shows initiating 304 an air purification cycle, directing 306 an airflow to an air inlet of an air filtration unit, with the air filtration unit including an air duct having a longitudinal axis, said air duct comprising an air inlet at a first end and an air outlet at a second end, a high efficiency particulate air (HEPA) filter unit oriented proximate to the air inlet, an airflow controller in communication with the air inlet, a carbon dioxide sensor in communication with the airflow controller, and a pressure sensor in communication with the airflow controller. The air filtration unit further includes an ultraviolet light reactor that further includes a plurality of baffles, with each baffle having a plurality of airflow spaces allowing airflow therethrough, disposed at spaced locations within the duct between the air inlet and air outlet, and with the baffles being generally transverse to the longitudinal axis. The air filtration unit further includes a plurality of ultraviolet light emitting diodes mounted on each baffle, a porous and permeable photocatalytic oxidation filter module disposed between each pair of baffles, generally transverse to the longitudinal axis, such that air flows through the photocatalytic oxidation filter module, and a porous and permeable nitrous oxide-adsorbing filter disposed downstream of the UV light reactor, and wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds. The method further includes removing 308 an amount of particulate from the airflow upstream from the photocatalytic oxidation filter modules, illuminating 310 the photocatalytic oxidation filter modules with ultraviolet light from ultraviolet light emitting diodes mounted on each baffle, chemically reducing 312 volatile organic compounds in the airflow to non-volatile organic compounds (thus removing VOCs from the airflow), and removing 314 an amount of nitrous oxide-containing compounds from the airflow downstream from the photocatalytic oxidation filter modules and wherein each photocatalytic oxidation filter module contains one or more catalysts comprising titanium dioxide (TiO₂), TiO₂ doped with iron (Fe-TiO₂), or TiO₂ doped with carbon (C-TiO₂) which, when illuminated by UV light, are operative to chemically reduce volatile organic compounds in the airflow to non-volatile organic compounds. Method 500, shown FIG. 11 further includes conducting 404 heat away from LEDs via at least one heat sink, with the at least one heat sink disposed within the duct and adapted to conduct heat away from the UV LEDs away and maintain the lifespan of the UV LEDs to, for example, prevent overheating of the UV LEDs and prolong the life of the UV LEDs (e.g., in a low airflow situation).

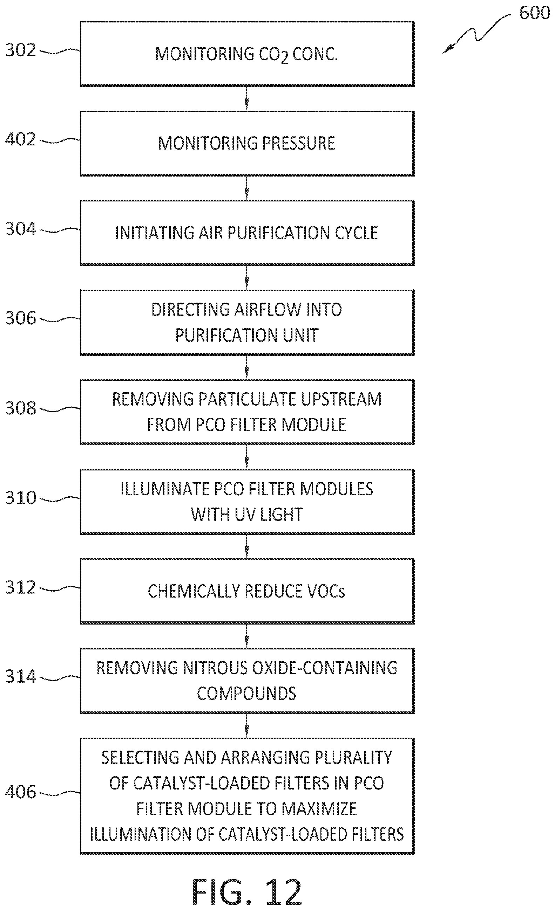

Figure 12 is a flowchart outlining a presently disclosed method for filtering air in an enclosed environment, with the method 600 including monitoring 302 carbon dioxide concentration in an enclosed environment, monitoring 402 pressure of the system for the purpose of sensing a pressure drop indicating that the need for adjusting a system airflow capacity, for example. The pressure can be monitored at an air intake or an air outlet, with the pressure monitored by a sensor that can send a signal to a controller that can adjust airflow capacity through the unit and system, etc.

Method 600 shown in Figure 12 further shows initiating 304 an air purification cycle, directing 306 an airflow to an air inlet of an air filtration unit, with the air filtration unit including an air duct having a longitudinal axis, said air duct comprising an air inlet at a first end and an air outlet at a second end, a high efficiency particulate air (HEPA) filter unit oriented proximate to the air inlet, an airflow controller in communication with the air inlet, a carbon dioxide sensor in communication with the airflow controller, and a pressure sensor in communication with the airflow controller. The air filtration unit further includes an ultraviolet light reactor that further includes a plurality of baffles, with each baffle having a plurality of airflow spaces allowing airflow therethrough, disposed at spaced locations within the duct between the air inlet and air outlet, and with the baffles being generally transverse to the longitudinal axis. The air filtration unit further includes a plurality of ultraviolet light emitting diodes mounted on each baffle, a porous and permeable photocatalytic oxidation filter module disposed between each pair of baffles, generally transverse to the longitudinal axis, such that air flows through the photocatalytic oxidation filter module, and a porous and permeable nitrous oxide-adsorbing filter disposed downstream of the UV light reactor, and wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds. The method further includes removing 308 an amount of particulate from the airflow upstream from the photocatalytic oxidation filter modules, illuminating 310 the photocatalytic oxidation filter modules with ultraviolet light from ultraviolet light emitting diodes mounted on each baffle, chemically reducing 312 volatile organic compounds in the airflow to non-volatile organic compounds (thus removing VOCs from the airflow), and removing 314 an amount of nitrous oxide-containing compounds from the airflow downstream from the photocatalytic oxidation filter modules and wherein each photocatalytic oxidation filter module contains one or more catalysts comprising titanium dioxide (TiO₂), TiO₂ doped with iron (Fe-TiO₂), or TiO₂ doped with carbon (C-TiO₂) which, when illuminated by UV light, are operative to chemically reduce volatile organic compounds in the airflow to non-volatile organic compounds. Method 600 further includes selecting and arranging 406 a plurality of catalyst-loaded filters in the photocatalytic oxidation (PCO) component in the VOC removal unit to maximize illumination of the catalyst-loaded filters.

The method 600, though not shown in Figure 12, can further include (as shown in method 500 shown in Figure 11) conducting 404 heat away from LEDs via at least one heat sink, with the at least one heat sink disposed within the duct and adapted to conduct heat away from the UV LEDs away and maintain the lifespan of the UV LEDs to, for example, prevent overheating of the UV LEDs and prolong the life of the UV LEDs (e.g., in a low airflow situation).

According to present aspects, the airflow rates of air delivered through the present air filtration units, and according to presently disclosed methods, can be, for example, from about 283.17 to about 424.75 l/m³ (about 10 to about 15 ft³/minute (CFM)) per occupant of purified airflow, with the understanding that the overall unit and system sizing and scale can be configured to accommodate and service enclosed environments (e.g., rooms, hallways, buildings, warehouses, garages, terrestrial transportation building environments, etc.).

Present apparatuses, systems, and methods are further understood to monitor, determine, and respond to CO₂ levels determined by the CO₂ sensors, and the system pressures observed, monitored, and detected by the pressure sensors. The combined factors of sensed CO₂ in an environment, and the changes in sensed CO₂ levels while the present systems are in operation, and further in view of sensed system pressures, can be relayed to one or more airflow controllers to adjust, in real time, airflow to be directed into the present systems.

The present disclosure can, of course, be carried out in other ways than those specifically set forth herein without departing from essential characteristics of the disclosure. The present aspects are to be considered in all respects as illustrative and not restrictive, and all changes coming within the range of the appended claims are intended to be embraced therein.

## Claims

1. An air filtration unit (10) comprising:
an air duct (14), said air duct comprising an air inlet (12) at a first end and an air outlet (16) at a second end;
a high efficiency particulate air filter (200) oriented proximate to the air inlet;
an airflow controller (202) in communication with the air inlet;
a carbon dioxide sensor (206) (206a) in communication with the airflow controller;
a pressure sensor (208) in communication with the airflow controller;
an ultraviolet light reactor (10a), the ultraviolet light reactor comprising; a plurality of baffles (18), each having a plurality of airflow spaces allowing airflow therethrough, disposed at spaced locations within the duct between the air inlet and air outlet, the baffles being generally transverse to the longitudinal axis;
a plurality of ultraviolet light emitting diodes (24) mounted on each baffle;
a porous and permeable photocatalytic oxidation filter module (30) disposed between each pair of baffles, generally transverse to the longitudinal axis, such that air flows through the photocatalytic oxidation filter module;
a porous and permeable nitrous oxide-adsorbing filter (210) disposed downstream of the ultraviolet light reactor; and
wherein each photocatalytic oxidation filter module contains one or more catalysts comprising titanium dioxide (TiO₂), TiO₂ doped with iron (Fe-TiO₂), TiO₂ doped with carbon (C-TiO₂), or combinations thereof which, when illuminated by ultraviolet light, are operative to chemically reduce volatile organic compounds to non-volatile organic compounds.

2. The air filtration unit (10) of Claim 1, wherein the carbon dioxide sensor (206) (206a) is in communication with at least one of the air inlet (12) and the air outlet (16), and/or wherein the pressure sensor (208) is in communication with at least one of the air inlet and the air outlet, and/or wherein the porous and permeable nitrous oxide-adsorbing filter (210) is oriented proximate to the air outlet (16).

3. The air filtration unit (10) of Claim 1 or 2, wherein the porous and permeable nitrous oxide-adsorbing filter (210) comprises a solid amine-containing adsorbent and/or a packed solid adsorbent, said packed solid adsorbent comprising at least one of: a cellular monolith; a granular media; a metal-organic containing compound; and zeolite.

4. The air filtration unit (10) of any of Claims 1-3, wherein one or more components of the air filtration unit (10), including one or more of the high efficiency particulate air filter, the ultraviolet light reactor (10a), the photocatalytic oxidation filter module (30), and the nitrous oxide-adsorbing filter (210), is configured to be integrated into the air filtration unit (10) as a discrete component that is configured to be individually removable from the air duct (14) and replaceable.

5. The air filtration unit (10) of any of Claims 1-4, further comprising one or more heat sink configured to be disposed within the duct, said one or more heat sink further adapted to conduct heat away from the ultraviolet light emitting diodes.

6. The air filtration unit (10) of any of Claims 1-5, wherein the ultraviolet light emitting diodes (24) are disposed both around a periphery of each baffle and between the airflow spaces, said ultraviolet light emitting diodes configured to maximize ultraviolet illumination of an adjacent photocatalytic oxidation filter module (30).

7. The air filtration unit (10) of any of Claims 1-6, wherein the photocatalytic oxidation filter module (30) is spaced apart from the baffles such that both surfaces of each photocatalytic oxidation filter module is illuminated by ultraviolet light.

8. The air filtration unit of any of Claims 1-7 wherein the photocatalytic oxidation filter module (30) comprises a plurality of filters, each filter including one or more of a coarse foam, a fine foam, a fused quartz filament felt, or combination thereof, and wherein each filter is loaded with a catalyst including one or more of pure titanium dioxide (TiO₂), TiO₂ doped with iron (Fe-TiO₂), TiO₂ doped with carbon (C-TiO₂), or combination thereof.

9. The air filtration unit (10) of any of Claims 1-8 wherein the ultraviolet light reactor comprises four baffles (18) and three photocatalytic oxidation filter modules, and wherein the three photocatalytic oxidation filter modules (30) comprise, in order from air inlet (12) to air outlet (16), the following configurations:
| | |
|---|---|
| module 1) | R25 - CTR - TA - R25; |
| module 2) | CTR - TA - Q25 - R25 - R25; and |
| module 3) | R25 - CTR - TA - R25, wherein |
R25 is a coarse foam loaded with pure TiO₂;
Q25 is a fused quartz filament felt loaded with pure TiO₂;
CTR is a coarse foam loaded with C-TiO₂ and
TA is a fine foam loaded with pure TiO₂.

10. The air filtration unit (10) of any of Claims 1-9, wherein the ultraviolet light reactor comprises four baffles and three photocatalytic oxidation filter modules (30), and wherein the photocatalytic oxidation filter modules comprise, in order from air inlet (12) to air outlet (16), the following configurations:
| | |
|---|---|
| module 1) | R25 - TA - FTR - CTR; |
| module 2) | R25 - CTA - FTR - Q25 - R25; and |
| module 3) | R25 - TA - FTR - CTR; wherein |
R25 is a coarse foam loaded with pure TiO₂;
Q25 is a fused quartz filament felt loaded with pure TiO₂;
CTR is a coarse foam loaded with C-TiO₂;
TA is a fine foam loaded with pure TiO₂; and
FTR is a coarse foam loaded with Fe-TiO₂.

11. A method (300) for filtering air in an enclosed environment, the method comprising:
monitoring (302) carbon dioxide concentration in an enclosed environment;
initiating an air purification cycle;
directing (304) an airflow to an air inlet (12) of an air filtration unit (200), said air filtration unit comprising:
an air duct (14) having a longitudinal axis, said air duct comprising an air inlet (12) at a first end and an air outlet (16) at a second end;
a high efficiency particulate air (HEPA) filter unit (204) oriented proximate to the air inlet;
an airflow controller (202) in communication with the air inlet;
a carbon dioxide sensor (206)(206a) in communication with the airflow controller;
a pressure sensor (208) in communication with the airflow controller;
an ultraviolet light reactor (10a), said ultraviolet light reactor comprising;
a plurality of baffles (18), each having a plurality of airflow spaces allowing airflow therethrough, disposed at spaced locations within the duct between the air inlet and air outlet, the baffles being generally transverse to the longitudinal axis;
a plurality of ultraviolet light emitting diodes (24) mounted on each baffle;
a porous and permeable photocatalytic oxidation filter module (30) disposed between each pair of baffles, generally transverse to the longitudinal axis, such that air flows through the photocatalytic oxidation filter module;
a porous and permeable nitrous oxide-adsorbing filter (210) disposed downstream of the ultraviolet light reactor (10a);
removing (308) an amount of particulate from the airflow upstream from the photocatalytic oxidation filter modules;
illuminating (310) the photocatalytic oxidation filter modules with ultraviolet light from ultraviolet light emitting diodes (24) mounted on each baffle;
chemically reducing (312) volatile organic compounds in the airflow to non-volatile organic compounds;
removing (314) an amount of nitrous oxide-containing compounds from the airflow downstream from the photocatalytic oxidation filter modules (30); and
wherein each photocatalytic oxidation filter module contains one or more catalysts comprising titanium dioxide (TiO₂), TiO₂ doped with iron (Fe-TiO₂), or TiO₂ doped with carbon (C-TiO₂) which, when illuminated by UV light, are operative to chemically reduce volatile organic compounds in the airflow to non-volatile organic compounds.

12. The method (300) of Claim 11, wherein the carbon dioxide sensor is in communication with at least one of the air inlet (12) and the air outlet (16).

13. The method (300) of Claim 11 or 12, wherein the porous and permeable nitrous oxide-adsorbing filter (210) is oriented proximate to the air outlet (16).

14. The method (300) of any of Claims 11-13 further comprising:
conducting heat from the ultraviolet light emitting diodes away from the ultraviolet light emitting diodes (24) via one or more heat sinks disposed within the duct.

15. The method (300) of any of Claims 11-14, wherein illuminating the photocatalytic oxidation filter modules with ultraviolet light further comprises:
selecting and arranging a plurality of catalyst-loaded filters to form each of said photocatalytic oxidation filter module (30) so as to maximize ultraviolet illumination of the plurality of catalyst-loaded filters.

## Patentansprüche

1. Luftfiltereinheit (10), umfassend:
einen Luftkanal (14), wobei der Luftkanal einen Lufteinlass (12) an einem ersten Ende und einen Luftauslass (16) an einem zweiten Ende umfasst;
einen hocheffizienten Partikelluftfilter (200), der in der Nähe des Lufteinlasses ausgerichtet ist;
einen Luftstromregler (202), der mit dem Lufteinlass in Verbindung steht;
einen Kohlendioxidsensor (206) (206a), der mit dem Luftstromregler in Verbindung steht;
einen Drucksensor (208), der mit dem Luftstromregler in Verbindung steht;
einen Ultraviolettlichtreaktor (10a), wobei der Ultraviolettlichtreaktor umfasst:
eine Vielzahl von Leitblechen (18), von denen jedes eine Vielzahl von Luftstromräumen aufweist, die einen Luftstrom durch sie hindurch ermöglichen, die an beabstandeten Stellen innerhalb des Kanals zwischen dem Lufteinlass und dem Luftauslass angeordnet sind, wobei die Leitbleche im Allgemeinen quer zur Längsachse verlaufen;
eine Vielzahl von Ultraviolett-Leuchtdioden (24), die auf jedem Leitblech montiert sind;
ein poröses und durchlässiges photokatalytisches Oxidationsfiltermodul (30), das zwischen jedem Paar von Leitblechen im Allgemeinen quer zur Längsachse angeordnet ist, so dass Luft durch das photokatalytische Oxidationsfiltermodul strömt;
einen porösen und durchlässigen Distickstoffoxid adsorbierenden Filter (210), der stromabwärts des Ultraviolettlichtreaktors angeordnet ist; und
wobei jedes photokatalytische Oxidationsfiltermodul einen oder mehrere Katalysatoren enthält, umfassend Titandioxid (TiO₂), TiO₂ dotiert mit Eisen (Fe-TiO₂), TiO₂ dotiert mit Kohlenstoff (C-TiO₂), oder Kombinationen davon, die bei Bestrahlung mit ultraviolettem Licht wirksam sind, flüchtige organische Verbindungen zu nichtflüchtigen organischen Verbindungen chemisch zu reduzieren.

2. Luftfiltereinheit (10) nach Anspruch 1, wobei der Kohlendioxidsensor (206) (206a) mit mindestens einem von dem Lufteinlass (12) und dem Luftauslass (16) in Verbindung steht, und/oder wobei der Drucksensor (208) mit mindestens einem von dem Lufteinlass und dem Luftauslass in Verbindung steht, und/oder wobei der poröse und durchlässige Distickstoffoxid adsorbierende Filter (210) in der Nähe des Luftauslasses (16) ausgerichtet ist.

3. Luftfiltereinheit (10) nach Anspruch 1 oder 2, wobei der poröse und durchlässige Distickstoffoxid adsorbierende Filter (210) ein festes, Amin enthaltendes Adsorbens und/oder ein gepacktes festes Adsorbens umfasst, wobei das gepackte feste Adsorbens mindestens eines von Folgendem umfasst: einen zellulären Monolith; ein körniges Medium; eine metallorganische Verbindung; und Zeolith.

4. Luftfiltereinheit (10) nach einem der Ansprüche 1-3, wobei eine oder mehrere Komponenten der Luftfiltereinheit (10), einschließlich eines oder mehrerer von dem hocheffizienten Partikelluftfilter, dem Ultraviolettlichtreaktor (10a), dem photokatalytischen Oxidationsfiltermodul (30), und dem Stickoxid adsorbierenden Filter (210), so konfiguriert ist, dass sie als eine diskrete Komponente in die Luftfiltereinheit (10) integriert wird, die so konfiguriert ist, dass sie einzeln aus dem Luftkanal (14) entfernbar und austauschbar ist.

5. Luftfiltereinheit (10) nach einem der Ansprüche 1-4, weiter umfassend einen oder mehrere Kühlkörper, die zum Anordnen innerhalb des Kanals konfiguriert sind, wobei der oder die Kühlkörper weiter dazu angepasst sind, Wärme von den ultravioletten Leuchtdioden abzuleiten.

6. Luftfiltereinheit (10) nach einem der Ansprüche 1-5, wobei die Ultraviolett-Leuchtdioden (24) sowohl um einen Umfang jedes Leitblechs herum als auch zwischen den Luftstromräumen angeordnet sind, wobei die Ultraviolett-Leuchtdioden dazu konfiguriert sind, die Ultraviolettbeleuchtung eines benachbarten photokatalytischen Oxidationsfiltermoduls (30) zu maximieren.

7. Luftfiltereinheit (10) nach einem der Ansprüche 1-6, wobei das photokatalytische Oxidationsfiltermodul (30) von den Leitblechen beabstandet ist, so dass beide Oberflächen jedes photokatalytischen Oxidationsfiltermoduls mit ultraviolettem Licht beleuchtet werden.

8. Luftfiltereinheit nach einem der Ansprüche 1-7, wobei das photokatalytische Oxidationsfiltermodul (30) eine Vielzahl von Filtern umfasst, wobei jeder Filter einen oder mehrerer von grobem Schaum, feinem Schaum, geschmolzenem Quarzfilamentfilz oder eine Kombination davon einschließt, und wobei jeder Filter mit einem Katalysator beladen ist, einschließend eines oder mehrere von reinem Titandioxid (TiO₂), TiO₂ dotiert mit Eisen (Fe-TiO₂), TiO₂ dotiert mit Kohlenstoff (C-TiO₂) oder eine Kombination davon.

9. Luftfiltereinheit (10) nach einem der Ansprüche 1-8, wobei der Ultraviolettlichtreaktor vier Leitbleche (18) und drei photokatalytische Oxidationsfiltermodule umfasst, und wobei die drei photokatalytischen Oxidationsfiltermodule (30) in der Reihenfolge vom Lufteinlass (12) zum Luftauslass (16) die folgenden Konfigurationen umfassen:
| | |
|---|---|
| Modul 1) | R25 - CTR - TA - R25; |
| Modul 2) | CTR - TA - Q25 - R25 - R25; und |
| Modul 3) | R25 - CTR - TA - R25, wobei |
R25 ein grober Schaum ist, der mit reinem TiO₂ beladen ist;
Q25 ein Quarzfilamentfilz ist, der mit reinem TiO₂ beladen ist;
CTR ein grober Schaum ist, der mit C-TiO₂ beladen ist, und
TA ein feiner Schaum ist, der mit reinem TiO₂ beladen ist.

10. Luftfiltereinheit (10) nach einem der Ansprüche 1-9, wobei der Ultraviolettlichtreaktor vier Leitbleche und drei photokatalytische Oxidationsfiltermodule (30) umfasst, und wobei die photokatalytischen Oxidationsfiltermodule in der Reihenfolge vom Lufteinlass (12) zum Luftauslass (16) die folgenden Konfigurationen umfassen:
| | |
|---|---|
| Modul 1) | R25 - TA - FTR - CTR; |
| Modul 2) | R25 - CTA - FTR - Q25 - R25; und |
| Modul 3) | R25 - TA - FTR - CTR; wobei |
R25 ein grober Schaum ist, der mit reinem TiO₂ beladen ist;
Q25 ein Quarzfilamentfilz ist, der mit reinem TiO₂ beladen ist;
CTR ein grober Schaum ist, der mit C-TiO₂ beladen ist;
TA ein feiner Schaum ist, der mit reinem TiO₂ beladen ist; und
FTR ein grober Schaum ist, der mit Fe-TiO₂ beladen ist.

11. Verfahren (300) zum Filtern von Luft in einer geschlossenen Umgebung, wobei das Verfahren umfasst:
Überwachen (302) der Kohlendioxidkonzentration in einer geschlossenen Umgebung;
Einleiten eines Luftreinigungszyklus;
Leiten (304) eines Luftstroms zu einem Lufteinlass (12) einer Luftfiltereinheit (200), wobei die Luftfiltereinheit umfasst:
einen Luftkanal (14), der eine Längsachse aufweist, wobei der Luftkanal einen Lufteinlass (12) an einem ersten Ende und einen Luftauslass (16) an einem zweiten Ende umfasst;
eine hocheffiziente Partikelfiltereinheit (HEPA) (204), die in der Nähe des Lufteinlasses ausgerichtet ist;
einen Luftstromregler (202), der mit dem Lufteinlass in Verbindung steht;
einen Kohlendioxidsensor (206)(206a), der mit dem Luftstromregler in Verbindung steht;
einen Drucksensor (208), der mit dem Luftstromregler in Verbindung steht;
einen Ultraviolettlichtreaktor (10a), wobei dieser Ultraviolettlichtreaktor umfasst:
eine Vielzahl von Leitblechen (18), von denen jedes eine Vielzahl von Luftstromräumen aufweist, die einen Luftstrom durch sie hindurch ermöglichen, die an beabstandeten Stellen innerhalb des Kanals zwischen dem Lufteinlass und dem Luftauslass angeordnet sind, wobei die Leitbleche im Allgemeinen quer zur Längsachse verlaufen;
eine Vielzahl von Ultraviolett-Leuchtdioden (24), die auf jedem Leitblech montiert sind;
ein poröses und durchlässiges photokatalytisches Oxidationsfiltermodul (30), das zwischen jedem Paar von Leitblechen im Allgemeinen quer zur Längsachse angeordnet ist, so dass Luft durch das photokatalytische Oxidationsfiltermodul strömt;
einen porösen und durchlässigen Distickstoffoxid adsorbierenden Filter (210), der stromabwärts des Ultraviolettlichtreaktors (10a) angeordnet ist;
Entfernen (308) einer Partikelmenge aus dem Luftstrom stromaufwärts der photokatalytischen Oxidationsfiltermodule;
Beleuchten (310) der photokatalytischen Oxidationsfiltermodule mit ultraviolettem Licht von Ultraviolett-Leuchtdioden (24), die an jedem Leitblech montiert sind;
chemisches Reduzieren (312) flüchtiger organischer Verbindungen im Luftstrom zu nichtflüchtigen organischen Verbindungen;
Entfernen (314) einer Menge an Distickstoffoxid enthaltenden Verbindungen aus dem Luftstrom stromabwärts der photokatalytischen Oxidationsfiltermodule (30); und
wobei jedes photokatalytische Oxidationsfiltermodul einen oder mehrere Katalysatoren enthält, umfassend Titandioxid (TiO₂), TiO₂ dotiert mit Eisen (Fe-TiO₂), oder TiO₂ dotiert mit Kohlenstoff (C-TiO₂), die bei Bestrahlung mit UV-Licht wirksam sind, flüchtige organische Verbindungen im Luftstrom chemisch zu nichtflüchtigen organischen Verbindungen reduzieren.

12. Verfahren (300) nach Anspruch 11, wobei der Kohlendioxidsensor mit mindestens einem von dem Lufteinlass (12) und dem Luftauslass (16) in Verbindung steht.

13. Verfahren (300) nach Anspruch 11 oder 12, wobei der poröse und durchlässige Distickstoffoxid adsorbierende Filter (210) in der Nähe des Luftauslasses (16) ausgerichtet ist.

14. Verfahren (300) nach einem der Ansprüche 11-13, weiter umfassend:
Ableiten von Wärme von den Ultraviolett-Leuchtdioden weg von den Ultraviolett-Leuchtdioden (24) über einen oder mehrere Kühlkörper, die innerhalb des Kanals angeordnet sind.

15. Verfahren (300) nach einem der Ansprüche 11-14, wobei das Beleuchten der photokatalytischen Oxidationsfiltermodule mit ultraviolettem Licht weiter umfasst:
Auswählen und Anordnen einer Vielzahl von mit Katalysator beladenen Filtern zum Bilden jedes der photokatalytischen Oxidationsfiltermodule (30), um eine ultraviolette Beleuchtung der Vielzahl von mit Katalysator beladenen Filtern zu maximieren.

## Revendications

1. Unité de filtration d'air (10) comprenant :
un conduit d'air (14), ledit conduit d'air comprenant une entrée d'air (12) au niveau d'une première extrémité et une sortie d'air (16) au niveau d'une seconde extrémité ;
un filtre (200) haute efficacité pour particules aériennes orienté à proximité de l'entrée d'air ;
un régulateur de flux d'air (202) en communication avec l'entrée d'air ;
un capteur de dioxyde de carbone (206) (206a) en communication avec le régulateur de flux d'air ;
un capteur de pression (208) en communication avec le régulateur de flux d'air ;
un réacteur à lumière ultraviolette (10a), le réacteur à lumière ultraviolette comprenant ;
une pluralité de déflecteurs (18), présentant chacun une pluralité d'espaces d'écoulement d'air permettant l'écoulement d'air à travers ceux-ci, disposés au niveau d'emplacements espacés à l'intérieur du conduit entre l'entrée d'air et la sortie d'air, les déflecteurs étant globalement transversaux à l'axe longitudinal ;
une pluralité de diodes électroluminescentes ultraviolettes (24) montées sur chaque déflecteur ;
un module (30) de filtre d'oxydation photocatalytique poreux et perméable disposé entre chaque paire de déflecteurs, globalement transversal à l'axe longitudinal, de telle sorte que l'air s'écoule à travers le module de filtre d'oxydation photocatalytique ;
un filtre (210) d'adsorption d'oxyde nitreux poreux et perméable disposé en aval du réacteur à lumière ultraviolette ; et
dans laquelle chaque module de filtre d'oxydation photocatalytique contient un ou plusieurs catalyseurs comprenant du dioxyde de titane (TiO₂), du TiO₂ dopé au fer (Fe-TiO₂), du TiO₂ dopé au carbone (C-TiO₂), ou des combinaisons de ceux-ci qui, lorsqu'ils sont éclairés par une lumière ultraviolette, sont efficaces pour réduire chimiquement les composés organiques volatils en composés organiques non volatils.

2. Unité de filtration d'air (10) selon la revendication 1, dans laquelle le capteur de dioxyde de carbone (206) (206a) est en communication avec au moins un élément parmi l'entrée d'air (12) et la sortie d'air (16), et/ou dans laquelle le capteur de pression (208) est en communication avec au moins un élément parmi l'entrée d'air et la sortie d'air, et/ou dans laquelle le filtre (210) d'adsorption d'oxyde nitreux poreux et perméable est orienté à proximité de la sortie d'air (16).

3. Unité de filtration d'air (10) selon la revendication 1 ou 2, dans laquelle le filtre (210) d'adsorption d'oxyde nitreux poreux et perméable comprend un adsorbant contenant des amines solides et/ou un adsorbant solide garni, ledit adsorbant solide garni comprenant au moins un élément parmi : un monolithe cellulaire ; un milieu granulaire ; un composé contenant des composés organométalliques ; et une zéolite.

4. Unité de filtration d'air (10) selon l'une quelconque des revendications 1 à 3, dans laquelle un ou plusieurs composants de l'unité de filtration d'air (10), incluant un ou plusieurs éléments parmi le filtre haute efficacité pour particules aériennes, le réacteur à lumière ultraviolette (10a), le module (30) de filtre d'oxydation photocatalytique, et le filtre (210) d'adsorption d'oxyde nitreux, est configuré pour être intégré dans l'unité de filtration d'air (10) sous la forme d'un composant discret qui est configuré pour être enlevable individuellement du conduit d'air (14) et remplaçable.

5. Unité de filtration d'air (10) selon l'une quelconque des revendications 1 à 4, comprenant en outre un ou plusieurs dissipateurs de chaleur configurés pour être disposés à l'intérieur du conduit, ledit un ou plusieurs dissipateurs de chaleur étant en outre adaptés à conduire la chaleur à distance des diodes électroluminescentes ultraviolettes.

6. Unité de filtration d'air (10) selon l'une quelconque des revendications 1 à 5, dans laquelle les diodes électroluminescentes ultraviolettes (24) sont disposées à la fois autour d'une périphérie de chaque déflecteur et entre les espaces d'écoulement d'air, lesdites diodes électroluminescentes ultraviolettes étant configurées pour maximiser l'éclairage ultraviolet d'un module (30) de filtre d'oxydation photocatalytique adjacent.

7. Unité de filtration d'air (10) selon l'une quelconque des revendications 1 à 6, dans laquelle le module (30) de filtre d'oxydation photocatalytique est espacé des déflecteurs de telle sorte que les deux surfaces de chaque module de filtre d'oxydation photocatalytique sont éclairées par la lumière ultraviolette.

8. Unité de filtration d'air selon l'une quelconque des revendications 1 à 7, dans laquelle le module (30) de filtre d'oxydation photocatalytique comprend une pluralité de filtres, chaque filtre incluant un ou plusieurs éléments parmi une mousse grossière, une mousse fine, un feutre de filaments de quartz fondu, ou une combinaison de ceux-ci, et dans laquelle chaque filtre est chargé d'un catalyseur incluant un ou plusieurs éléments parmi du dioxyde de titane pur (TiO₂), du TiO₂ dopé au fer (Fe-TiO₂), du TiO₂ dopé au carbone (C-TiO₂), ou une combinaison de ceux-ci.

9. Unité de filtration d'air (10) selon l'une quelconque des revendications 1 à 8, dans laquelle le réacteur à lumière ultraviolette comprend quatre déflecteurs (18) et trois modules de filtre d'oxydation photocatalytique, et dans laquelle les trois modules (30) de filtre d'oxydation photocatalytique comprennent, dans l'ordre depuis l'entrée d'air (12) vers la sortie d'air (16), les configurations suivantes :
| | |
|---|---|
| module 1) | R25 - CTR - TA - R25 ; |
| module 2) | CTR - TA - Q25 - R25 - R25 ; et |
| module 3) | R25 - CTR - TA - R25, dans laquelle |
R25 est une mousse grossière chargée de TiO₂ pur ;
Q25 est un feutre de filaments de quartz fondu chargé de TiO₂ pur ;
CTR est une mousse grossière chargée de C-TiO₂ et
TA est une mousse fine chargée de TiO₂ pur.

10. Unité de filtration d'air (10) selon l'une quelconque des revendications 1 à 9, dans laquelle le réacteur à lumière ultraviolette comprend quatre déflecteurs et trois modules (30) de filtre d'oxydation photocatalytique, et dans laquelle les modules de filtre d'oxydation photocatalytique comprennent, dans l'ordre depuis l'entrée d'air (12) vers la sortie d'air (16), les configurations suivantes :
| | |
|---|---|
| module 1) | R25 - TA - FTR - CTR ; |
| module 2) | R25 - CTA - FTR - Q25 - R25 ; et |
| module 3) | R25 - TA - FTR - CTR; dans laquelle |
R25 est une mousse grossière chargée de TiO₂ pur ;
Q25 est un feutre de filaments de quartz fondu chargé de TiO₂ pur ;
CTR est une mousse grossière chargée de C-TiO₂ ;
TA est une mousse fine chargée de TiO₂ pur ; et
FTR est une mousse grossière chargée de Fe-TiO₂.

11. Procédé (300) destiné à filtrer l'air dans un environnement clos, le procédé comprenant :
surveiller (302) la concentration de dioxyde de carbone dans un environnement clos ;
initier un cycle de purification d'air ;
diriger (304) un écoulement d'air vers une entrée d'air (12) d'une unité de filtration d'air (200), ladite unité de filtration d'air comprenant :
un conduit d'air (14) présentant un axe longitudinal, ledit conduit d'air comprenant une entrée d'air (12) au niveau d'une première extrémité et une sortie d'air (16) au niveau d'une seconde extrémité ;
une unité de filtre haute efficacité pour particules aériennes (HEPA, high efficiency particulate air) (204) orientée à proximité de l'entrée d'air ;
un régulateur de flux d'air (202) en communication avec l'entrée d'air ;
un capteur de dioxyde de carbone (206) (206a) en communication avec le régulateur de flux d'air ;
un capteur de pression (208) en communication avec le régulateur de flux d'air ;
un réacteur à lumière ultraviolette (10a), ledit réacteur à lumière ultraviolette comprenant ;
une pluralité de déflecteurs (18), présentant chacun une pluralité d'espaces d'écoulement d'air permettant l'écoulement d'air à travers ceux-ci, disposés au niveau d'emplacements espacés à l'intérieur du conduit entre l'entrée d'air et la sortie d'air, les déflecteurs étant globalement transversaux à l'axe longitudinal ;
une pluralité de diodes électroluminescentes ultraviolettes (24) montées sur chaque déflecteur ;
un module (30) de filtre d'oxydation photocatalytique poreux et perméable disposé entre chaque paire de déflecteurs, globalement transversal à l'axe longitudinal, de telle sorte que l'air s'écoule à travers le module de filtre d'oxydation photocatalytique ;
un filtre (210) d'adsorption d'oxyde nitreux poreux et perméable disposé en aval du réacteur à lumière ultraviolette (10a) ;
éliminer (308) une quantité de particules en provenance de l'écoulement d'air en amont des modules de filtre d'oxydation photocatalytique ;
éclairer (310) les modules de filtre d'oxydation photocatalytique par la lumière ultraviolette en provenance des diodes électroluminescentes ultraviolettes (24) montées sur chaque déflecteur ;
réduire chimiquement (312) les composés organiques volatils dans l'écoulement d'air en composés organiques non volatils ;
éliminer (314) une quantité de composés contenant de l'oxyde nitreux en provenance de l'écoulement d'air en aval des modules (30) de filtre d'oxydation photocatalytique ; et
dans lequel chaque module de filtre d'oxydation photocatalytique contient un ou plusieurs catalyseurs comprenant du dioxyde de titane (TiO₂), TiO₂ dopé au fer (Fe-TiO₂), ou du TiO₂ dopé au carbone (C-TiO₂) qui, lorsqu'ils sont éclairés par une lumière ultraviolette, sont efficaces pour réduire chimiquement les composés organiques volatils dans l'écoulement d'air en composés organiques non volatils.

12. Procédé (300) selon la revendication 11, dans lequel le capteur de dioxyde de carbone est en communication avec au moins un élément parmi l'entrée d'air (12) et la sortie d'air (16).

13. Procédé (300) selon la revendication 11 ou 12, dans laquelle le filtre (210) d'adsorption d'oxyde nitreux poreux et perméable est orienté à proximité de la sortie d'air (16).

14. Procédé (300) selon l'une quelconque des revendications 11 à 13 comprenant en outre :
conduire la chaleur en provenance des diodes électroluminescentes ultraviolettes à distance des diodes électroluminescentes ultraviolettes (24) par le biais d'un ou plusieurs dissipateurs de chaleur disposés à l'intérieur du conduit.

15. Procédé (300) selon l'une quelconque des revendications 11 à 14, dans lequel l'éclairage des modules de filtre d'oxydation photocatalytique par la lumière ultraviolette comprend en outre :
choisir et agencer une pluralité de filtres chargés en catalyseur pour former chacun desdits modules (30) de filtre d'oxydation photocatalytique de manière à maximiser l'éclairage ultraviolet de la pluralité de filtres chargés en catalyseur.
